Europäisches Patentamt

European Patent Office

Office européen des brevets

(19)

(11) Publication number : **0 451 981 A2**

# (12) EUROPEAN PATENT APPLICATION

(21) Application number : 91302542.5

(22) Date of filing : 22.03.91

(51) Int. Cl.⁵ : **G01N 33/66, A61B 5/00, C12Q 1/54**

(30) Priority : 26.03.90 US 499187

(43) Date of publication of application :
16.10.91 Bulletin 91/42

(84) Designated Contracting States :
AT BE CH DE DK ES FR GB GR IT LI LU NL SE

(71) Applicant : CASCADE MEDICAL, INC.
10180 Viking Drive
Eden Prairie, Minnesota 55344 (US)

(72) Inventor : Anderson, Paul J.
8243 Thomas Avenue South
Bloomington, Minnsota 55431 (US)

Inventor : Carlson, Thomas C.
6907 Sand Ridge Road
Eden Prairie, Minnesota 55346 (US)
Inventor : Jessen, Ross A.
305 Waters Edge Drive
Chaska, Minnesota 55318 (US)
Inventor : Matuska, Dennis D.
1901 East 14th Street
Glencoe, Minnesota 55336 (US)
Inventor : Jones, Richard E.
7901 Rhode Island Circle
Bloomington, Minnesota 55438 (US)
Inventor : Walter, Bert
809 Park Place Drive
Mendota Heights, Minnesota 55118 (US)

(74) Representative : Parr, Ronald Edward R.E. Parr
& Co.
Colman House Station Road
Knowle Solihull West Midlands B93 0HL (GB)

(54) Disposable reagent unit.

(57) A disposable reagent unit including a base strip with a plurality of spaced holes, a double face tape, a reagent and a lot identification paper in a side-by-side adjacent relationship, a wick, and a cover strip with a hole for engagement with a diagnostic instrument quantitative of an analyte in a liquid such as glucose in blood. Blood is deposited in the blood bowl and is wicked to the reagent for subsequent color change other detectable change to be measured by a diagnostic instrument. The color intensify of the reagent is proportional to the analyte such as glucose in the blood.

EP 0 451 981 A2

FIG. 5

## BACKGROUND OF THE INVENTION

1. Field of the Invention - The present invention pertains to a test device for use in the analysis of a liquid or for measuring a component quality or quantity of a liquid. The invention relates particularly, but not exclusively, to the medical field, for example to disposable diagnostic reagent unit for sampling and analyzing blood or any components of blood for specific readings as to qualities of the blood. One specific use of the present invention is for sensing the accumulating of blood glucose in diabetics. In a preferred form the invention relates to a disposable diagnostic reagent unit for use with a medical diagnostic system. In one form, such system is used with a disposable lancet.

A diagnostic system in which, or with which, the disposable diagnostic reagent unit or other test device of the present invention can be used is described and claimed in a European application filed on the same day as the present application and by the same applicant under the title "Medical Diagnostic System". That European application claims priority from US Patent Application 07/499 085 filed 26 March 1990.

The invention is described hereinafter with particular reference to the analysis of blood with reference to its glucose content but applies, mutatis mutandis, to the analysis of analytes in liquids generally.

2. Description of the Prior Art - One representative prior art Patent is US Patent No. 4 787 398, issued on November 29, 1988, to Garcia et al., for "Glucose Medical Monitoring System", which discloses a disposable reagent unit with a lancet.

In one form the present invention provides a disposable reagent unit without a lancet for use in a diagnostic instrument, for example a medical diagnostic instrument.

## SUMMARY OF THE INVENTION

The general purpose of the present invention is a multi-laminate disposable reagent test device for quantitative measurement of analyte in fluid with the use of a hand-held instrument. The test device contains an analytical component, a fluid transport component and an electronic detectable lot-correction component.

According to one embodiment of the present invention, there is provided a disposable reagent test device including a base strip with a plurality of ports, lot-identification paper and adjacent reagent material, a wick, and a cover strip with at least one port, where the base strip and the cover strip are suitably secured together, for example by mechanical riveting, glue or adhesive.

Preferred embodiments of the present invention have one or more of the following features and advantages:

(a) a disposable reagent unit which can be readily manufactured and packaged in a sterile foil package, and is leak-proof when utilized with a liquid;

(b) a reagent unit which can include one or more reagent windows, and an optional reagent specific calibration window;

(c) a reagent strip which can be used to measure quantity of at least one analyte in a liquid;

(d) measurement of glucose and cholesterol in blood;

(e) a reagent strip that engages in a diagnostic instrument which reads resultant color change of the reagent indicating the quantity of at least one component such as glucose in a liquid such as blood;

(f) a disposable reagent unit to measure a predetermined analyte in a liquid; and

(g) a multi-laminate disposable reagent unit which is leak-proof for purposes of infection control and for purposes of disposal.

There are now described, by way of example and with reference to the accompanying drawings, preferred embodiments of the present invention.

In the drawings:

**FIG. 1** illustrates a bottom view of a reagent strip;

**FIG. 2** illustrates a side view of the reagent strip;

**FIG. 3** illustrates a top view of a reagent strip;

**FIG. 4** illustrates an exploded side view of the reagent strip;

**FIG. 5** illustrates an exploded isometric view of the reagent strip;

**FIG. 6** illustrates use of the reagent strip with a diagnostic instrument;

**FIGS. 7A and 7B** illustrate a first alternative embodiment of a bottom view and a top view of a reagent strip;

**FIGS. 8A and 8B** illustrate a second alternative embodiment of a bottom view and a top view of a reagent strip.

**FIGS. 9A and 9B** illustrate a third alternative embodiment of a bottom view and top view of a reagent strip.

## DESCRIPTION OF THE PREFERRED EMBODIMENTS

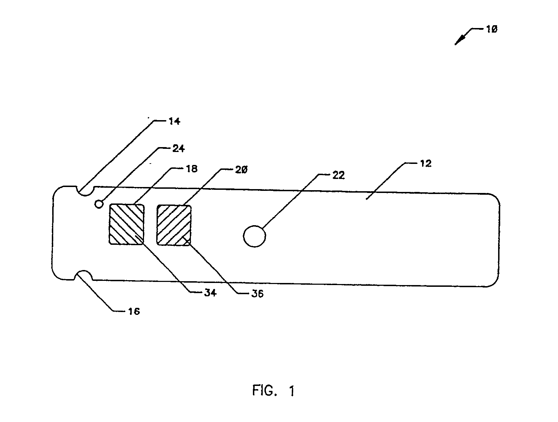

FIG. 1 illustrates a bottom view of a multi-laminate reagent strip 10 including base strip 12 with a plurality of notches, grooves or holes 14 and 16 (for air holes and gripping grooves), a reagent specific calibration window on a lot-to-lot identification window 18, a reagent window 20, a lancet port hole 22, and a vent hole 24. Any geometrical aperture can be utilized in lieu of the holes, ports, openings or windows. The multi-laminate structure forms a housing for the reagent unit.

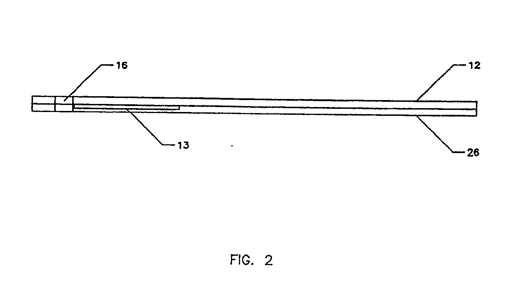

FIG. 2 illustrates a side view of the reagent strip 10 including a base strip 12 and a cover strip 26. An optional recess 13 is for accommodation of materials such as any reagent materials and lot-to-lot or calibration materials.

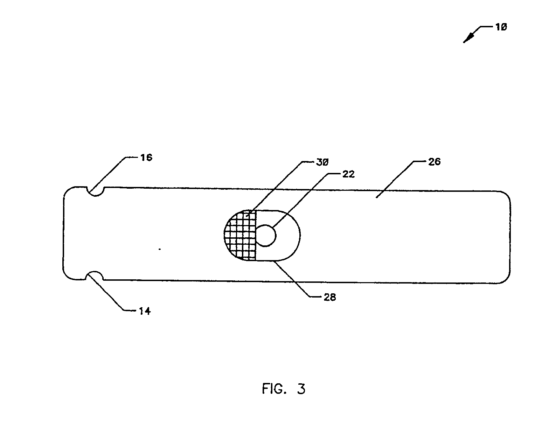

FIG. 3 illustrates a top view of the reagent strip 10 including a blood bowl 28 and a wick 30. The bowl 28 can be directly above the reagent or remove from the reagent. The transport of fluid is by capillary action, either by the wicking material which can be symmetric or asymmetric or by an air gap. The wicking material can also act as a filter. The vent hole (24) can be adjacent to or spaced with respect to the transporting structure for the liquid. The reagent material can be a"switchable"membrane such as preformed material such as polymer material, a film or a fiber such as paper or cloth material. The lot-to-lot identification port(18) can utilise optical detection (for example a gray scale or a color scale) a bar code or other optical code, magnetic detection, resistively detection, an EPROM with software, or any mechanical detection schemes such as with notches, etc., electrical detection schemes or electromechanical detection schemes. The lot identification port 18 is also optically used to detect a sufficient fluid sample and for reagent material lot programming. The strip housing can include extruded plastic, molded plastic, or milled plastic. The disposal reagent unit is multi-laminate. At least one port is provided for detecting analyte; fo example and two or more ports can be provided for detecting analyte. More than one reagent can be provided for detecting more than one analyte such as for a blood panel of tests. The reagent unit can be utilized with diagnostic units configured to analyze multi-reagents such as for glucose, cholesterol, alcohol, or any other analytes in blood, liquid or fluids.

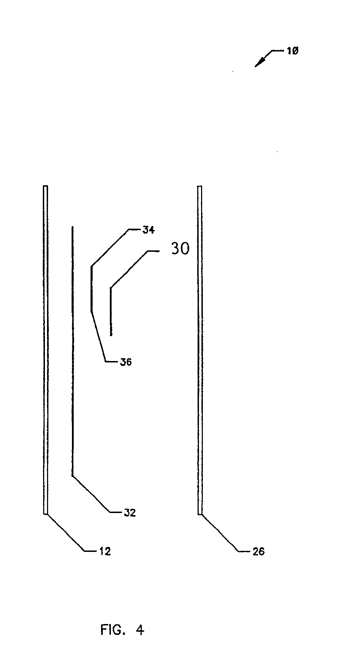

FIG. 4 illustrates an exploded side view of the reagent strip 10 including a base strip 12, a double face adhesive tape 32, a lot-to-lot paper 34, a reagent 36, a wick 30 and the cover strip 26.

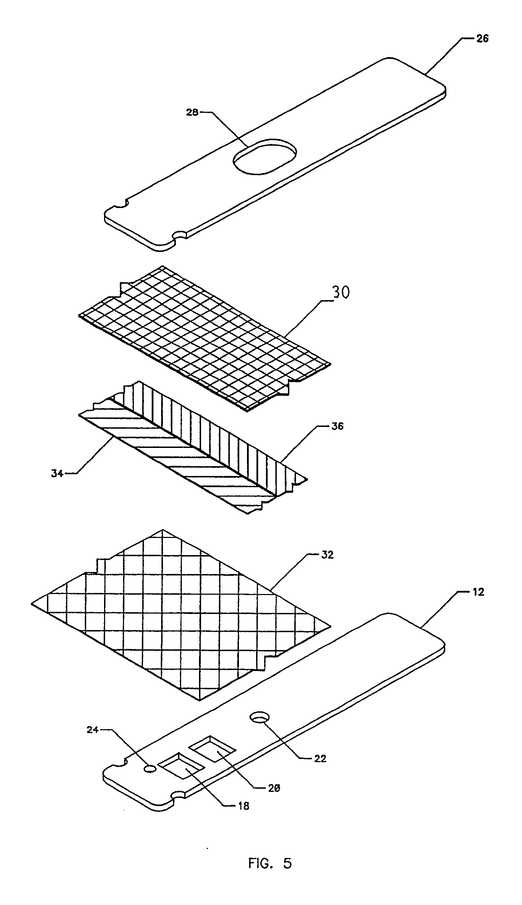

FIG. 5 illustrates an exploded isometric view of the reagent strip where all numerals correspond to those elements previously described.

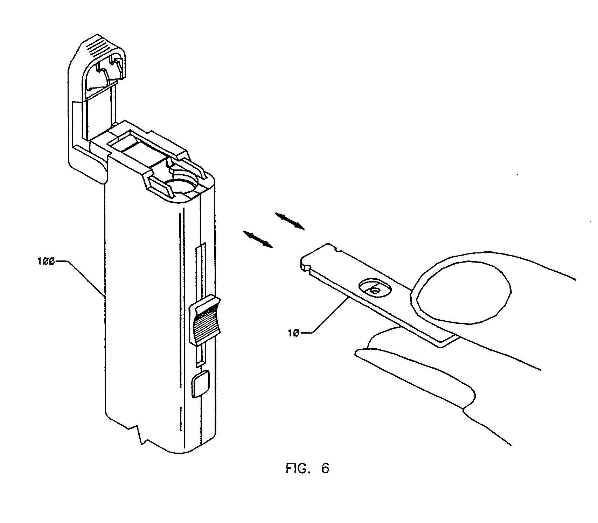

FIG. 6 illustrates use of the reagent strip 10 in use with a diagnostic instrument 100.

## MODE OF OPERATION

The blood glucose reagent strips are designed to be used with a reflectance meter for quantitative determination of glucose in whole blood. The glucose reagent employs a glucose oxidase/peroxidase chemistry in a dry reagent membrane enclosed in a black plastic housing. The disposable reagent unit features and functions includes:

a. Blood Bowl(28) - Blood application site that houses wick(30) and lancet hole (22).

b. Wick (30) - Transports blood sample from the blood bowl (28) to the chemistry.

c. Lancet hole (22) - Opening through which lancet tip emerges when triggered by the instrument.

d. Reagent Window (20) - Site of chemical reaction with glucose.

e. Lot-to-Lot Programming Window - Used for automatic programming of the appropriate instrument calibration and includes a calibration material. Other terms of definition include reagent specific calibration window and can also be used for sensing sufficient analyte as a secondary function.

f. Vent hole (24) - Opening to facilitate blood flow through the disposable reagent unit.

A drop of blood from a finger stick is applied to the white wick (30) material located in the oval opening (28) in the center of the disposable reagent unit. The wick transports the sample to the chemistry to initiate the reaction and helps compartmentalize the blood sample for safe and easy disposal.

The disposable reagent unit provides a simple and convenient means to measure blood glucose. With the reagent strip, no washing, wiping, blotting, timing or calibration steps are required.

The chemistry of the disposable reagent unit includes a containment plastic housing that contains a nylon reagent membrane with a glucose oxidase/peroxidase chemistry, and the indicator O-Tolidine.

When a blood sample contacts the reagent membrane, the glucose, in the presence of oxygen, is converted to- Gluconolactone and hydrogen peroxide by the enzyme glucose oxidase (GOD) (Reaction 1).

The enzyme peroxidase (POD) catalyzes the reaction between the indicator (beige) and hydrogen peroxide to produce water and an oxidized indicator (blue) (Reaction 2).

$$\text{Glucose} + O_2 \underline{\text{GOD}} \text{-Gluconolactone} + H_2O_2 \quad (1)$$
$$\text{O-Tolidine}_{(red)} + \underline{H_2O_2 \text{ POD}} \text{ O-Tolidine}_{(ox)} + 2H_2O \quad (2)$$

The intensity of the color produced is proportional to the sample glucose concentration.

The reagents are in Table 1 as followed by way of example:

<u>Table 1</u>

```
O-tolidine . . . . . . . . .. .23ug
Glucose Oxidase. . . . . . . 2.8 I.U.
Peroxidase . . . . . . . . .2.4 I.U.
Buffer (Stabilizers) . . . .13.7 ug
Inert Ingredients. . . . . .2630 ug
```

To perform a blood glucose test, a drop of blood can be obtained from a finger puncture using the lancing system built into the blood glucose monitor. Alternatively, fresh venous blood collected in EDTA or heparin containing tubes may also be used. To minimize glycolysis, test blood samples as close as possible (10 minutes or less) to the time the samples were collected

Glucose concentration of venous and capillary bloods may differ as much as 10 percent depending on the time of sample collection after a meal.

The system results are displayed as milligrams of glucose per deciliter of whole blood (mg/dL). Blood glucose levels associated with well-controlled diabetes are:

Fasting: 60-130 mg/dL blood glucose

After meals (1 hour): less than 180 mg/dL

Blood glucose monitoring and interpretation of results should be done with the guidance of a diabetes health care professional. A physician would be consulted to establish target blood glucose values that are suitable, insulin dose or other medication resulting from interpretation of glucose readings.

## DESCRIPTION OF THE ALTERNATIVE EMBODIMENTS

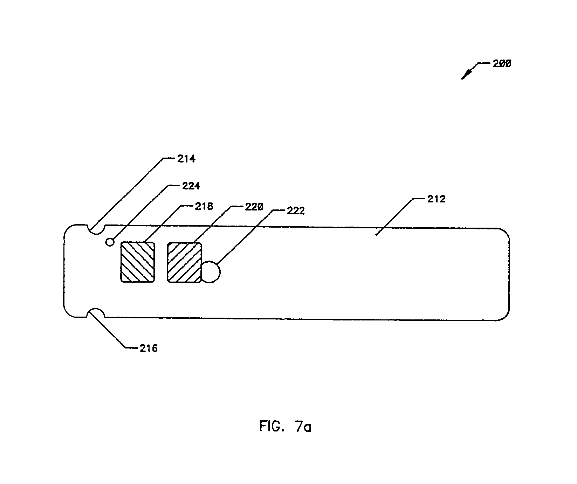

**FIG. 7A** illustrates a first alternative embodiment of a bottom view of a reagent strip 200, where the elements have been located at positions differing from the reagent strip 10, and along and about the base strip 212 and the cover strip 226, as illustrated. The reagent strip 200 includes elements similar in structure, name and function to the reagent strip 10 of Fig. 1. The base strip 212 includes a plurality of holes 214 and 216 for air holes and gripping grooves, a lot-to-lot identification window 218, a reagent window 220, a lancet port 222 and a vent hole 224.

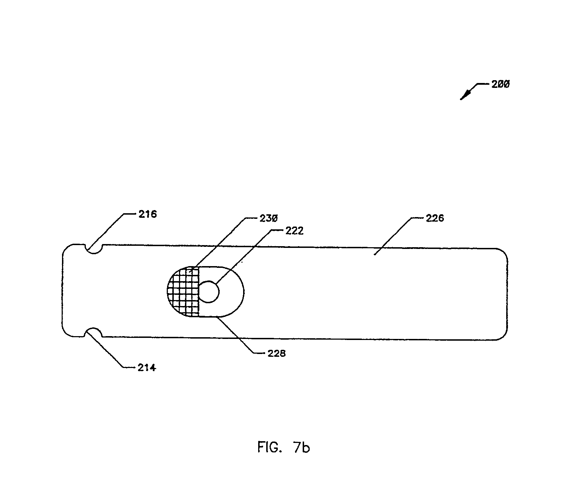

**FIG. 7B** illustrates a top view of the reagent strip 200 where all numerals correspond to those elements previously described. The cover strip 226 includes the blood bowl 228 and the wick 230. The blood bowl 228 is aligned substantially opposite the reagent window 220 on the opposing side of the base strip 212.

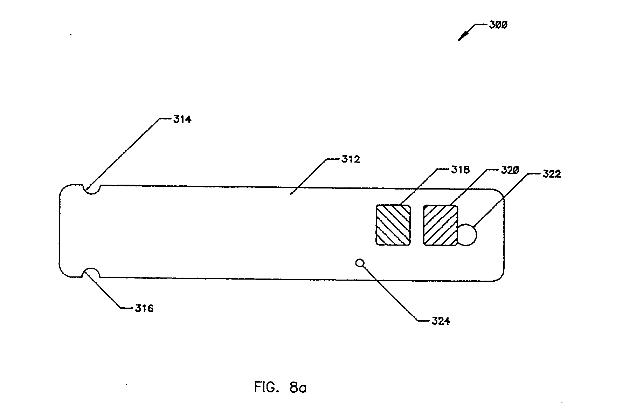

**FIG. 8A** illustrates a second alternative embodiment of a bottom view of a reagent strip 300 where the elements have been located at positions differing from the reagent strip 10, and along and about the base strip 312 and the cover strip 326, as illustrated. The reagent strip 300 includes elements similar in structure and function to the reagent strip 10 of Fig. 1. The base strip 312 includes a plurality of holes 314 and 316, for air holes and gripping grooves, a lot-to-lot identification window 318, a reagent window 320, a lancet port 322 and a vent hole 324. The lot-to-lot identification window 318, reagent window 320, the lancet hole 322 and the vent hole 324, are located in close proximity to the end of the reagent strip 200, which is opposite the end with the plurality of holes and grooves 314 and 316.

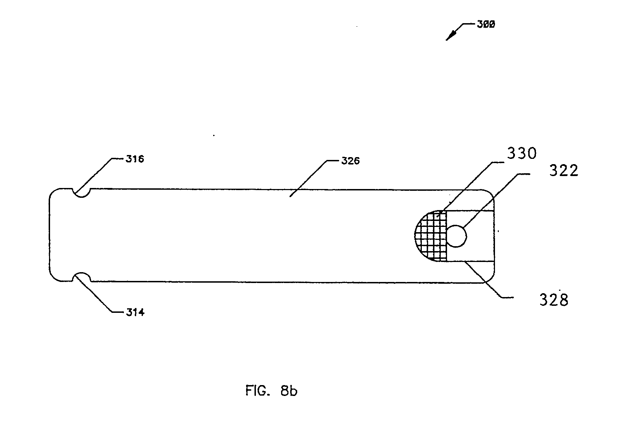

**FIG. 8B** illustrates a top view of the reagent strip 300 where all numerals correspond to those elements previously described. The cover strip 326 includes the blood bowl 328 and the wick 330. The blood bowl 328, is aligned opposite the reagent window 320 on the opposite side of the base strip 312 and adjacent to the edge of the reagent strip.

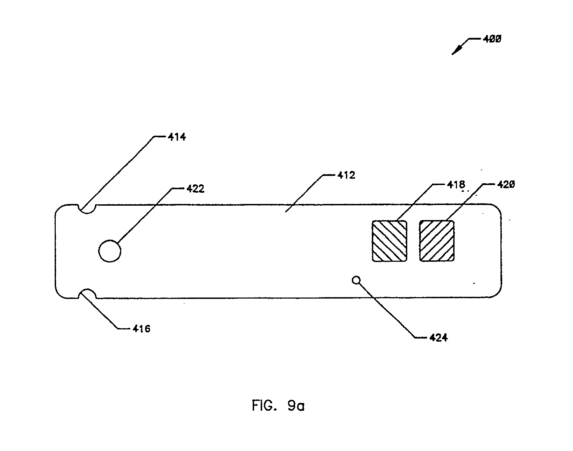

**FIG. 9A** illustrates a third alternative embodiment of a bottom view of a reagent strip 400 where the elements have been located at positions differing from the reagent strip 10, and along and about the base strip 412 and the cover strip 426, as illustrated. The reagent strip 400 includes elements similar in structure and function to the reagent strip 10 of Fig. 1. The base strip 412 includes a plurality of holes 414 and 416 for air vent holes and for gripping grooves, a lot-to-lot identification window 418, a reagent window 420, a spaced lancet port 422 , and a vent hole 424. The lot-to-lot identification window 418 , reagent window 420, and the vent hole 424, are located in close proximity to the end of the reagent strip 400 , which is opposite the end with the plurality of holes and grooves 414 and 416.

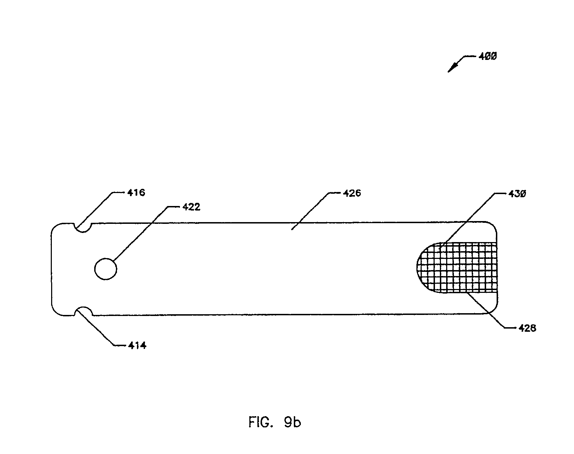

**FIG. 9B** illustrates a top view of the reagent strip 400 where all numerals correspond to those elements previously described. The cover strip 426 includes the blood bowl 428 and the wick 430 . The blood bowl 428, is aligned opposite the reagent window 420 on the opposite side of the base strip 412 and adjacent to the edge of the reagent strip.

More than one reagent window or one calibration window can be provided on the strip housing or multi-laminate structure. The size, arrangement and spacing of the windows, holes, and components is determined by the diagnostic system which utilizes the reagent strip. The reagent strip is intended for any system for measuring an analyte and is not limited strictly to a medical system as other uses of the reagent strip are applicable.

Although the invention has been described with particular reference to the use of "strips" to constitute the housing of the test device, those members can be any suitable housing member, for example in the form of plate or other sheet whose shape can be square or other rectangle, circular or other suitable shape. Where the test device is intended for use with any of the systems described in the European application referred to above that claims priority from US Application 07/499 085, it is usually convenient for the housing to comprise elongated rectangular members, usually planar.

Embodiments of the test device of the invention are as follows:

A. A multi-laminate test device comprising:
    a. a housing means for transportation and containment of a fluid;
    b. at least one sample application port in said housing means;
    c. a fluid transport means in said housing means; and
    d. a reagent means in said housing means for indication by a change of said reagent the presence of an analyte in said fluid.

B. A disposable reagent unit comprising in order:
    a. a first strip (26) including a first hole and at least one sample application port (28);
    b. a reagent material (36) over said application port (28):
    c. a transport means (30) communicating between said sample port and said reagent material; and
    d. a second strip (12) including a corresponding second hole over said first hole.

C. A disposable reagent unit comprising in order:
    a. a base strip (12) including a first sample hole, a hole for lot-to-lot material and a hole (20) for reagent material;
    b. a lot-to-lot material over said lot-to-lot hole;
    c. a reagent material (36) over said reagent hole;
    d. a wicking material (30) from said blood hole (28) to said reagent material and lot-to-lot hole; and
    e. a cover strip (26) including a corresponding second sample hole over said first sample hole, including means (32) securing said strips together.

Optional features of the invention which are of particular interest are as follows:
– said housing includes opposed, substantially parallel first and second upper and lower portions.
– there are at least two reagent means.
– said opening for providing a liquid substance to said reagent means includes said first opening.
– includes calibration means supported in an aperture in said housing next to said reagent means.

Abbreviations used herein have the following meanings:

o-Tolidine     - o-toluidine
EDTA     - ethylenediamine tetra-acetic acid
chemistry     - a chemical reagent or an indicator or signal means which operates in a chemical manner

One purpose of the lot-to-lot identification window, where present, is for differences in the manufacturing of material and calibrates the system prior to reading the reagent.

## Claims

1. A test device characterised in that it comprises:

    a housing comprising first and second sheets (26, 12) secured together in a superimposed disposition;

    said first sheet (26) having a port (28) therein to receive a liquid test sample containing an analyte to be tested;

    a reagent (36) disposed between the first and second sheets and selected to produce a desired response to the analyte;

    a conduit (30) to permit the liquid to pass from the port (28) to the reagent (36); and

    said second sheet (12) having a window or other display means (20) through which or by which said response can be observed or otherwise detected either qualitatively or quantitatively.

2. A test device according to Claim 1, wherein said desired response is a change of color or other visible signal.

3. A test device according to Claim 1 or 2, wherein the liquid sample comprises blood and the analyte is glucose.

4. A test device according to Claim 1, 2 or 3, wherein said second sheet (12) has a second window or other display means (18) to effect a calibration of the device.

5. A disposable diagnostic reagent unit for operative engagement with a medical monitoring diagnostic system, said disposable unit comprising:
   a. a housing (12, 26);
   b. means (14, 16) for operatively connecting said housing to said system;
   c. blood reagent chemistry means (36) supported within said housing;
   d. liquid-conveying means (30) including a first opening (28) in said housing for conveying a liquid substance to said reagent means; and
   e. aperture means (18) through said housing adjacent the system positioned to pass reflected light from at least a portion of said reagent means in said diagnostic unit to a sensing means in the system for a determination of change of said reagent means by the system whereby said change indicates a diagnostic condition.

6. A diagnostic reagent unit for operative engagement with a monitoring diagnostic system, said disposable unit comprising:
   a housing (12, 26) ;
   means (14, 16) for operatively connecting said housing to said system;
   blood reagent chemistry means (36) supported within said housing;
   liquid conveying means (30) including a first opening (28) in said housing for conveying a liquid substance to said reagent means; and
   aperture means (18) in the housing, the nature and disposition of the aperture with respect to the housing being such that, when the reagent unit is in said operative engagement with the monitoring system, the aperture can pass reflected light from at least a portion of said reagent means to a sensing means in the system for a determination of change of said reagent means by the system whereby said change indicates a diagnostic condition.

7. A disposable diagnostic reagent unit for operating in engagement and use with a medical diagnostic system of a type having a puncturing means, connection means for operatively receiving said unit on said system and means in said system for reading a detectable change on the reagent means, said disposable diagnostic reagent unit comprising:
   a. a housing;
   b. means on said housing for operatively connecting said housing to said system;
   c. reagent means including a front side facing said system and a back side away from said system, supported within said housing;
   d. liquid conveying means including a first opening in said housing for conveying a liquid substance from the punctured surface to said reagent means; and,
   e. aperture means through said housing at a point adjacent said system unit for conveying light from a back side portion of said reagent means to said system for determination of change thereby indicating a diagnostic condition.

8. A disposable diagnostic reagent unit according to Claim 7, wherein said surface is skin and said liquid substance is blood.

9. A disposable diagnostic reagent unit according to Claim 7 or 8, wherein the color change of said reagent is caused by glucose in said liquid substance and said diagnostic condition is glucose level.

10. A disposable diagnostic reagent unit for operating in engagement and use with a medical diagnostic system of a type having actuation means for driving of a puncturing means, connection means for operatively receiving said disposable reagent unit on said system and means in said system for reading a color change on the reagent means, said disposable diagnostic reagent unit comprising:

    a. a housing including opposing finger gripping tabs and a bottom plate;

    b. at least one reagent means having a variable color responsive to the concentration of an analyte in a liquid supported within said housing;

    c. liquid conveying means including said first opening in said housing for conveying said liquid from said punctured surface to said reagent means; and,

    d. aperture means through said housing for providing access for reading said reagent means for change of color by said system thereby indicating a diagnostic condition.

11. A reagent unit according to any of Claims 5 to 10, wherein said liquid-conveying means comprises a wick.

12. A reagent unit according to any of Claims 5 to 10, wherein said liquid-conveying means comprises a capillary air gap.

13. A reagent unit according to any of Claims 5 to 12, wherein said liquid-conveying means includes a filter for filtering at least one component from said liquid substance.

14. A reagent unit according to any of Claims 5 to 13, wherein the housing has a lot-to-lot calibration means.

FIG. 1

FIG. 2

FIG. 3

FIG. 4

FIG. 5

FIG. 6

FIG. 7a

200

216 230 222 226

228

214

FIG. 7b

FIG. 8a

300

316

326

330

322

314

328

FIG. 8b

FIG. 9a

FIG. 9b